# EUROPEAN PATENT APPLICATION

(11) **EP 4 549 916 A1**
(43) Date of publication of application: **07.05.2025**
(21) Application number: 24209048.8
(22) Date of filing: 25.10.2024
(51) Int. Cl.: G01N 21/78, G01N 21/84, G01N 21/93, G01N 21/01

(54) **MEASUREMENT SYSTEM, MEASUREMENT METHOD, MEASUREMENT PROGRAM, AND SMART DEVICE**

(30) Priority: 30.10.2023 JP 2023185916
(71) Applicant: ARKRAY, Inc., Kyoto-shi, Kyoto 601-8045 (JP)
(72) Inventor: UMENO, Naoki, Kyoto, 602-0008 (JP); HIRAMURA, Fumito, Kyoto, 602-0008 (JP)
(74) Representative: Dehns

(57) **Abstract**

A measurement system (10) that includes: a test strip (60) including an introduction portion (63) at an upstream side into which a sample is introduced, a measurement portion (72) at a downstream side on which a reaction reagent that reacts with a measurement target contained in the sample is immobilized, and a developing portion (73) for spreading the sample from the introduction portion (63) to downstream of the measurement portion (72); a light source (42) radiating light of a predetermined wavelength onto the test strip (60) into which the sample is introduced; an imaging section (51) acquiring optical information of the test strip (60) irradiated by the light source (42); and an analysis section (250) determining a type of the sample from the optical information of an upstream region (74) located at an upstream side in the developing portion (73) from among the optical information acquired by the imaging section (51).

## Description

### BACKGROUND

### Technical Field

The present invention relates to technology, in a system to measure a measurement target in a sample using a test strip, to identify a type of a sample to be used prior to the measurement.

### Related Art

Japanese Patent Application Laid-Open (JP-A) No. 2003-004743 discloses technology to distinguish a type of a test liquid from a detection signal in a cloth portion on a chromatography test strip to which a test liquid is added.

A calibration curve used in sample measurement varies according to whether a sample containing blood cell components (whole blood sample), or a sample not containing blood cell components (blood plasma or blood serum), is employed as the sample. Therefore, it is necessary to input a type of the sample to be spotted onto a test strip, to a measurement device in advance. Moreover, measurement is sometimes performed with a calibration curve of the wrong type when spot application is performed without confirming the type of the sample. Furthermore, correct measurement is sometimes not able to be performed until a whole blood sample reaches a measurement position. Thus, an exemplary embodiment of the present invention accordingly provides technology to identify in advance of measurement a type of a sample to be used in a system to measure a measurement target in a sample using a test strip.

### SUMMARY

A measurement system of an aspect of the present invention includes: a test strip including an introduction portion at an upstream side to which a sample is introduced, a measurement portion at a downstream side on which a reaction reagent that reacts with a measurement target contained in the sample is immobilized, and a developing portion for spreading the sample from the introduction portion to downstream of the measurement portion; a light source radiating light of a predetermined wavelength onto the test strip into which the sample is introduced; an imaging section acquiring optical information of the test strip irradiated by the light source; and an analysis section determining a type of the sample from optical information of an upstream region located at an upstream side in the developing portion from among the optical information acquired by the imaging section.

An exemplary embodiment of the present invention provides technology to identify, in advance of measurement, a type of a sample to be used in a system to measure a measurement target in a sample using a test strip.

### BRIEF DESCRIPTION OF THE DRAWINGS

Exemplary embodiments will be described in detail based on the following figures, wherein:
Fig. 1 is an upper perspective view of a holder employed in an exemplary embodiment;
Fig. 2 is a lower perspective view illustrating an enlargement of a vicinity of an insertion port of a holder;
Fig. 3A is a plan view of a test strip employed in an exemplary embodiment;
Fig. 3B illustrates a simplified test strip of Fig. 3A;
Fig. 3C schematically illustrates a structure of the test paper of Fig. 3A;
Fig. 4 is an upper perspective view of a state in which a test strip is mounted to a holder;
Fig. 5 is a plan view of the state in Fig. 4;
Fig. 6 is an upper perspective view of a placement section employed in an exemplary embodiment;
Fig. 7 is a bottom view of a placement section;
Fig. 8 is an upper perspective view of a housing of an exemplary embodiment;
Fig. 9 is an upper perspective view of a state in which a test strip is mounted to the housing of Fig. 8;
Fig. 10 is a bottom view of a smart device employed in an exemplary embodiment;
Fig. 11 is an upper perspective view of a measurement system of an exemplary embodiment;
Fig. 12 is an upper perspective view of a state in which part of an external wall section is removed from the measurement system of Fig. 11;
Fig. 13 illustrates a cross-section taken along XIII-XIII of Fig. 12;
Fig. 14 is a functional block diagram of a measurement system of an exemplary embodiment;
Fig. 15 illustrates a block diagram of a controller;
Fig. 16A is a flowchart illustrating an overview of a measurement method of a measurement target in a measurement system of an exemplary embodiment;
Fig. 16B is a flowchart illustrating an overview of a measurement method of a measurement target in a measurement system of an exemplary embodiment;
Fig. 17 is a graph illustrating an example of fluorescent light intensity in a developing portion when a whole blood sample is measured as a sample; and
Fig. 18 is a graph illustrating an example of fluorescent light intensity in a developing portion when blood plasma is measured as a sample.

### DETAILED DESCRIPTION

Description follows regarding exemplary embodiments of the present invention, with reference to the drawings. Common reference numerals across the drawings indicate same parts, unless explicitly stated otherwise. Moreover, each member and each site as represented in the drawings are each merely schematic representations thereof, and sizes and positional relationships of an actual product are not necessarily accurately represented therein.

### (1) Holder

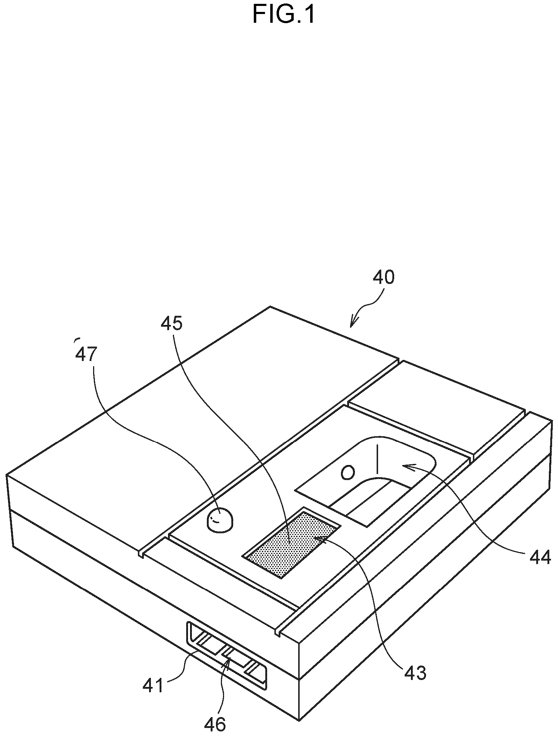

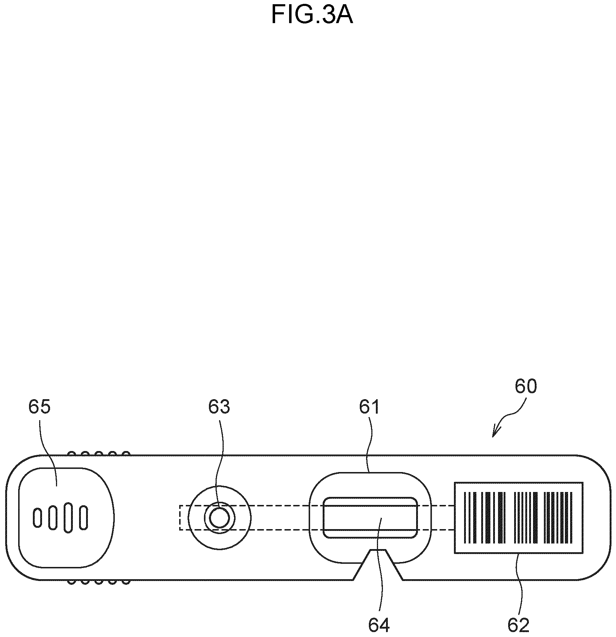

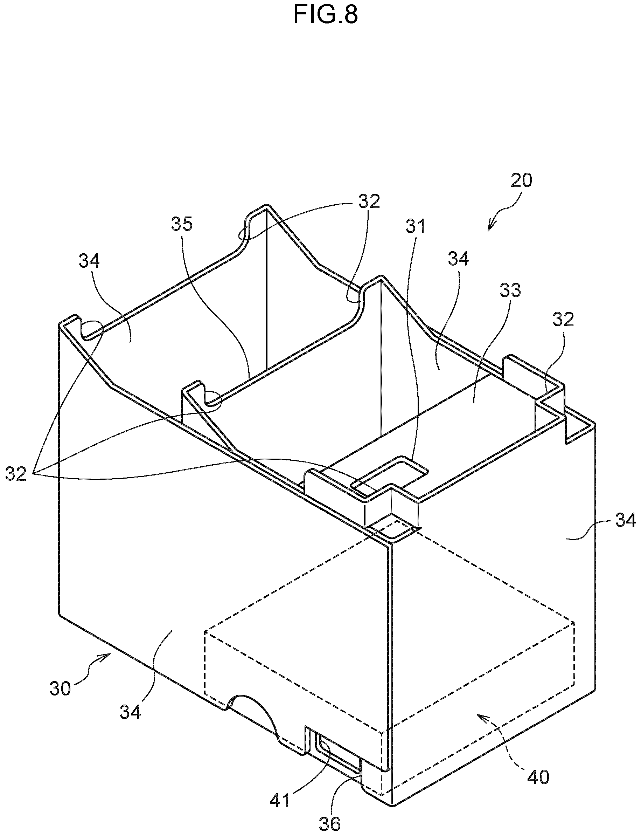

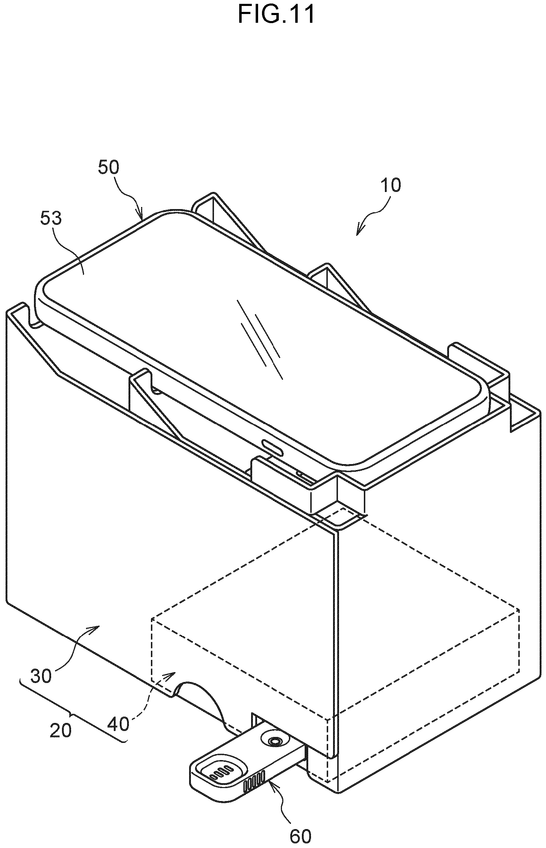

Fig. 1 is an upper perspective view of a holder 40 employed in a measurement system 10 of the present exemplary embodiment (see Fig. 11). The measurement system 10 may include a holder 40 such as illustrated in Fig. 1 as a member configuring a housing 20 (see Fig. 8). The holder 40 exhibits a box shape and is formed with two openings, i.e., a measurement opening section 43 and an identification opening section 44, in an upper face of the holder 40. An optical filter 45 is fitted into the measurement opening section 43. A sensor 47 for sensing light is furthermore provided to the upper face of the holder 40. An insertion port 41 into which a test strip 60 described later (see Fig. 3A) is to be inserted opens onto a side face of the holder 40. The interior space of the insertion port 41 is a space in communication with both the measurement opening section 43 and the identification opening section 44, and configures (provides) a housing section 46 that is a space in which a part of the test strip 60 is housed.

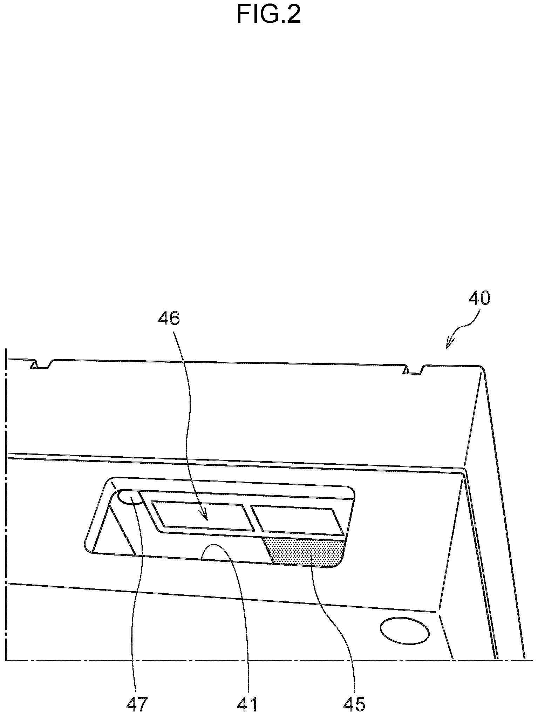

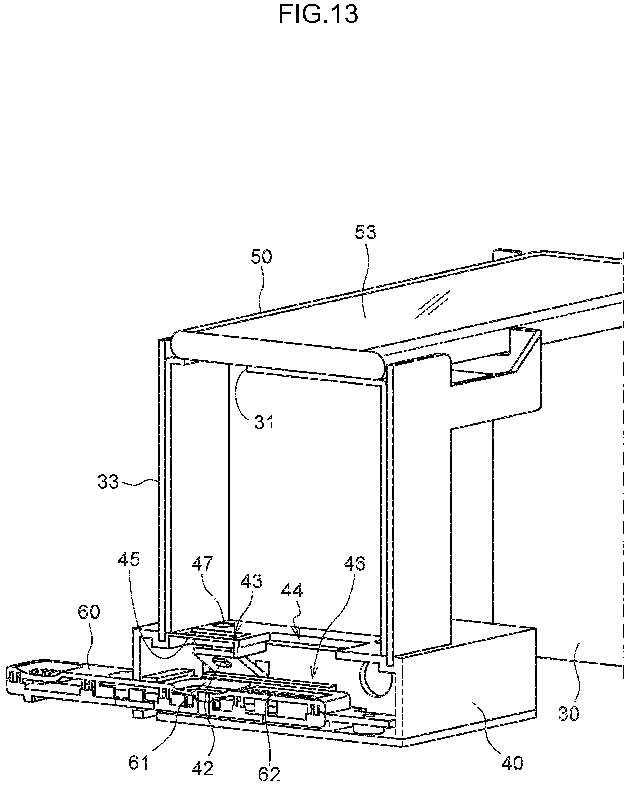

Fig. 2 is a lower perspective view illustrating an enlargement of a vicinity of the insertion port 41 of the holder 40. The sensor 47 is provided in the vicinity of the measurement opening section 43 (see Fig. 1) into which the optical filter 45 is fitted, in the housing section 46 which is an interior space in communication with the insertion port 41. Light rays of a wavelength appropriate for imaging a measurement region 61 are radiated from a light source 42 (see Fig. 13) provided inside the housing section 46. Then, the optical filter 45 has optical characteristics that suitably transmit only light rays of the wavelength appropriate for such imaging.

### (2) Test Strip

Fig. 3A illustrates a plan view of the test strip 60 employed in the measurement system 10 of the present exemplary embodiment (see Fig. 11). The test strip 60 exhibits a flat bar shaped external profile. A grip portion 65 whose upper face side is indented is formed to one end of the test strip 60, and the test strip 60 can be gripped by pinching the grip portion 65 with finger tips. A test paper 64 having a shape elongated along the length direction of the test strip 60 is housed inside the test strip 60. The test paper 64 is exposed toward above at two openings formed in the upper face of the test strip 60. These two openings include an introduction portion 63 near the grip portion 65, and a measurement region 61 at which measurement of the measurement target is performed further away from the grip portion 65. Furthermore, an identification region 62, which is recorded with information related to the test strip 60, is formed at the other end side of the test strip 60, namely, on the upper face of a location furthest away from the grip portion 65. Hereafter a side of the test strip 60 near to the grip portion 65 will be called an "upstream side", and a side thereof near to the identification region 62 will be called a "downstream side".

The test paper 64 is a water absorbent body such as filter paper or is configured by coating a water absorbent layer onto a surface of a synthetic resin substrate. A reaction reagent that reacts with the measurement target to develop a color is applied to an intermediate portion of the measurement region 61 on the test paper 64. A sample suspected to contain the measurement target is spotted onto the introduction portion 63. Examples of samples that may be employed in the present invention include, for example, a sample containing a blood cell component (hereafter referred to as a "whole blood sample") or a sample not containing a blood cell component (e.g., blood plasma or blood serum, hereafter referred to as a "non-blood cell sample"), or a diluted sample resulting from diluting either of these with an appropriate buffer.

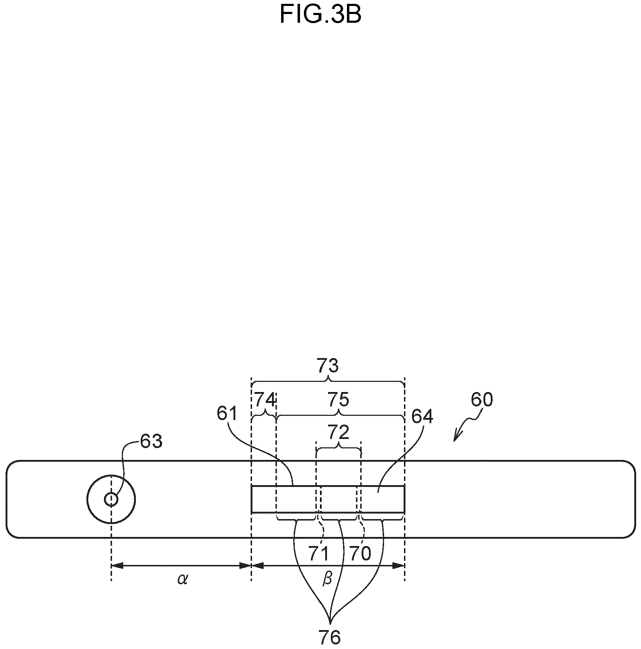

Fig. 3B illustrates a simplified test strip 60 of Fig. 3A, for ease of explanation. Namely, configuration other than the introduction portion 63 and the measurement region 61 is omitted therein. On the test paper 64, a portion visible in the measurement region 61 (see Fig. 3A) is a developing portion 73 along which a sample spreads from the upstream side to the downstream side. The developing portion 73 is divided into an upstream region 74 that is a region at the upstream side, and a comparison region 75 that is a region other therefrom, which are involved in the identification of the type of the sample, as described later. An intermediate portion of the comparison region 75 is a measurement portion 72 on which the reagent that reacts with a substance contained in the sample is immobilized.

Specifically, a reaction reagent that reacts specifically with the measurement target is immobilized in a downstream side portion of the measurement portion 72. A target reaction band 70 is generated by a reaction of the measurement target with the reaction reagent. Moreover, a control reagent that non-specifically reacts with the sample, irrespective of the presence or absence of the measurement target, is immobilized at an upstream side portion of the measurement portion 72. A control reaction band 71 is generated by a reaction of the sample with the control reagent. Irrespective of the presence or absence of the measurement target, correct spreading of the sample is indicated by the generation of the control reaction band 71. Namely, the developing portion 73 includes a region at which the target reaction band 70 is generated due to a specific reaction with the measurement target contained in the sample, and a region at which the control reaction band 71 is generated by a non-specific reaction with the sample. Then, a region in the comparison region 76 other than the region at which the target reaction band 70 is generated and the region at which the control reaction band 71 is configured is referred to as a standard region 76.

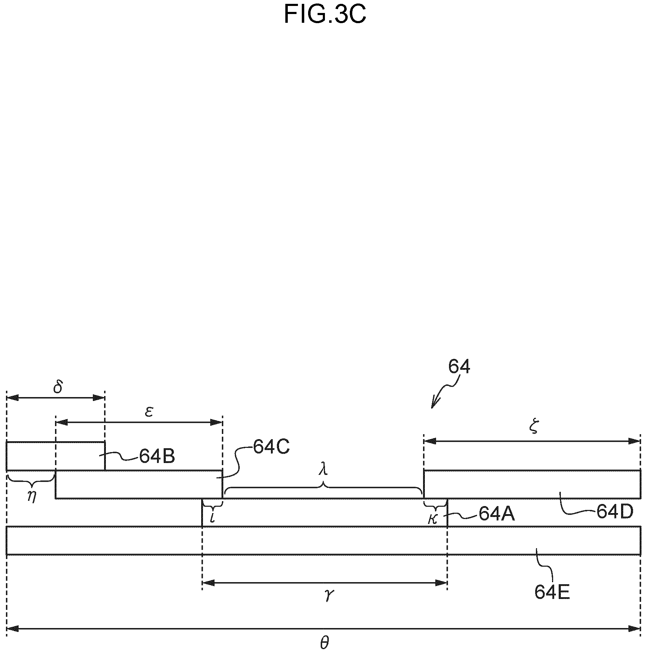

Fig. 3C schematically illustrates a structure of the test paper 64 of Fig. 3A. The test paper 64 has a development membrane 64A having a length encompassing the measurement region 61 (see Fig. 3B) fixed to an intermediate portion of a backing sheet 64E having a length spanning substantially the entire length of the test strip 60. The backing sheet 64E is formed from a material that does not transmit moisture such as, for example, a polyvinyl chloride (PVC) resin or a polystyrene (PS) resin. The development membrane 64A is able to absorb a liquid by capillary action, and is formed with a material capable of immobilizing the reaction reagent and the control reagent, for example with a material such as a nitrocellulose membrane.

A sample pad 64B is fixed at the upstream side of the development membrane 64A through a conjugate pad 64C. The sample pad 64B is positioned directly below the introduction portion 63 (see Fig. 3B). The sample pad 64B exhibits (comprises) a mesh-like structure, and has a function to trap a portion of blood cell components contained in a sample, and to let a blood plasma component thereof be transmitted downstream together with any blood cell components not trapped. The blood cell component transmitted through the sample pad 64B contributes, as described later, to identification as to whether the sample is a whole blood cell sample or a non-blood cell sample. The sample pad 64B is formed by a material such as, for example, glass fibers, polyethylene terephthalate (PET) resin or a cellulose fibers.

The conjugate pad 64C has a function to let a sample transmitted through the sample pad 64B be transmitted to the development membrane 64A. A function to trap blood cells, such as the sample pad 64B, may, or may not, be imparted to the conjugate pad 64C. Namely, a desired blood cell trapping function may be configured so as to be realized by a combination of the sample pad 64B and the conjugate pad 64C. The conjugate pad 64C is formed from a material such as, for example, glass fibers, polyester fibers, or rayon.

An absorption pad 64D is fixed to a downstream side of the development membrane 64A. The absorption pad 64D has a function to absorb excess liquid that has reached the downstream end of the development membrane 64A. The absorption pad 64D is capable of absorbing a liquid by capillary action, and is formed from a material such as, for example, glass fibers, cellulose fibers, or cotton.

When a sample is spotted onto the introduction portion 63, the sample permeates to the conjugate pad 64C through the sample pad 64B, and reaches the development membrane 64A. The sample spread downstream by the development membrane 64A reacts with the reagent in the measurement portion 72 (see Fig. 3B), and a target reaction band 70 is generated when the measurement target is present, and the control reaction band 71 is generated irrespective of the presence or absence of the measurement target. Here, in cases in which the sample contains blood cells, blood cells that reaches the development membrane 64A can be confined to the upstream region 74 by appropriately adjusting a blood cell trap function of the sample pad 64B (and/or the conjugate pad 64C), liquid permeation speed of the development membrane 64A, the size of each portion of the test strip 60, and the diluted concentration of the sample. Then, blood cell components that are confined to the upstream region 74 are then detected as optical information, and a type of the sample, specifically whether a whole blood sample or a non-blood cell sample, is identified from the detected optical information of the upstream region 74. This point is described further below.

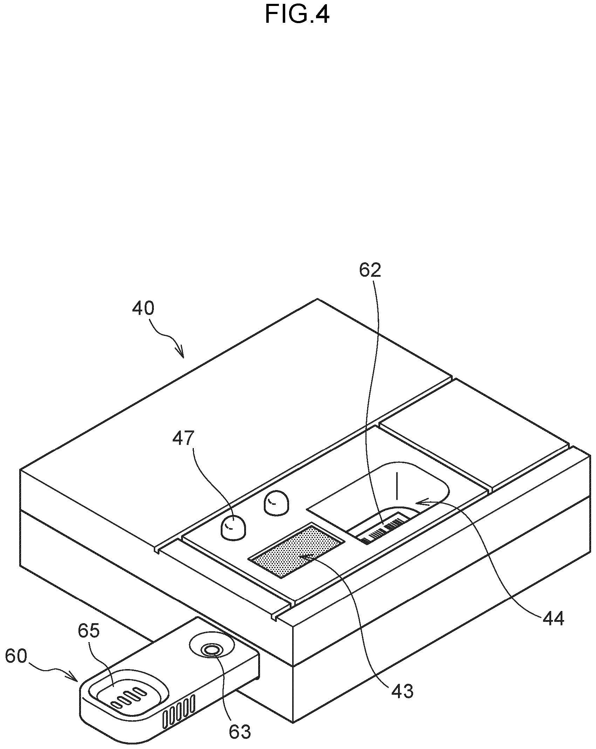

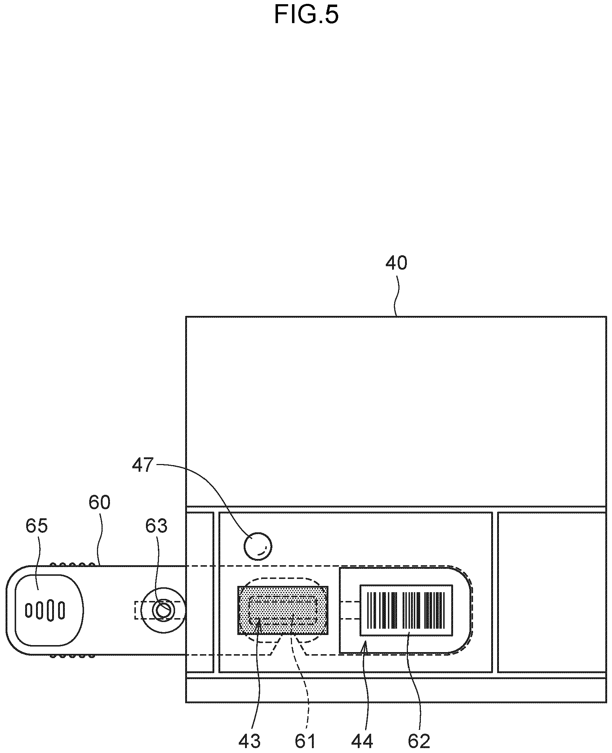

Fig. 4 is an upper perspective view illustrating a state in which the test strip 60 is mounted to the holder 40. Moreover, Fig. 5 is a plan view illustrating this state. As illustrated in Fig. 4 and Fig. 5, the test strip 60 is inserted downstream side first through the insertion port 41 and into the interior of the housing section 46 (see Fig. 1). In this state, as illustrated in Fig. 5, the measurement region 61 (see Fig. 3A) is positioned in the same flat plane as the measurement opening section 43, and, moreover, the identification region 62 is positioned in the same flat plane as the identification opening section 44.

In this state, when a sample is spotted onto the introduction portion 63, the sample flows downstream under a capillary action of the test paper 64, such that the control reaction band 71 (see Fig. 3B) indicating that the sample is spotted in the measurement region 61 occurs near the upstream side. Furthermore, in cases in which the measurement target is contained in the sample, the target reaction band 70 (see Fig. 3B) occurs near the downstream side having an intensity according to the concentration of the measurement target. In the test paper 64, the control reaction band 71 is positioned at a central portion of the measurement region 61, and the target reaction band 70 is positioned at the downstream side, outside the central portion. Light of a predetermined wavelength emitted from the light source 42 (see Fig. 13) is radiated onto the target reaction band 70, and the measurement system 10 of the present exemplary embodiment measures the concentration of the measurement target by measuring the intensity of light generated therefrom. The identification region 62 is recorded with identification information that is information related to the test strip 60, for example, which type of test paper 64 is housed in the test strip 60. Examples of the identification information include a bar code, a QR code (registered trademark), or the like.

### (3) Placement Section

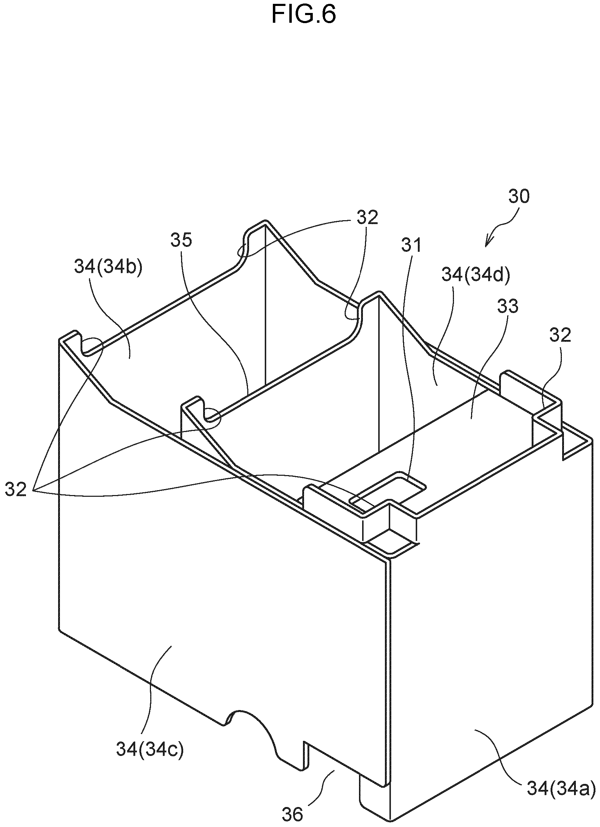

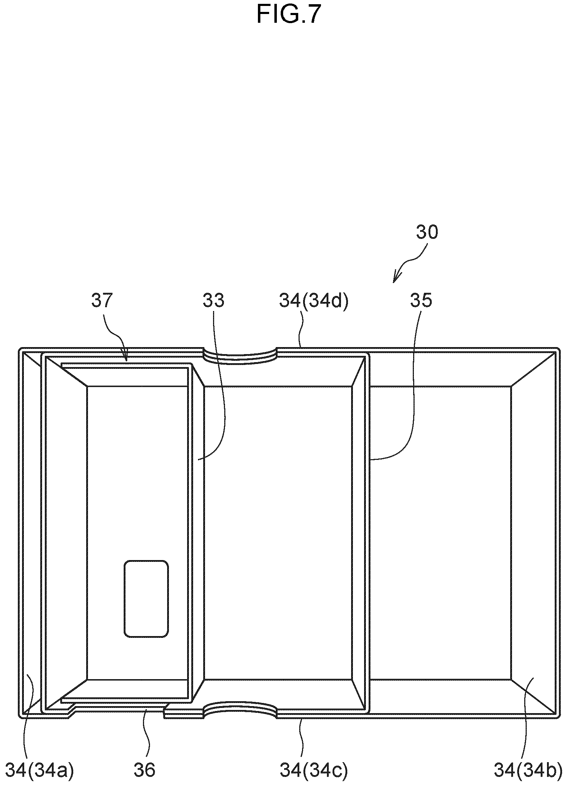

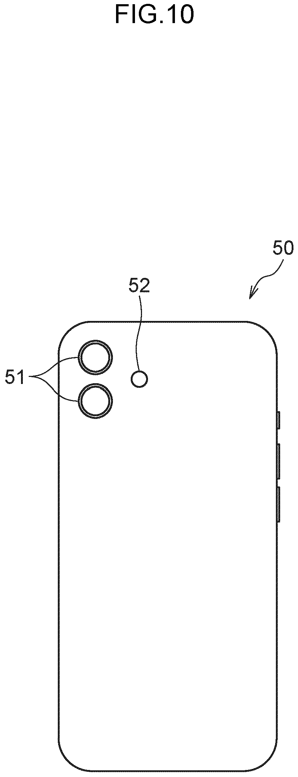

Fig. 6 is an upper perspective view including a placement section 30 employed in the measurement system 10 of the present exemplary embodiment (see Fig. 11). Moreover, Fig. 7 is a bottom view of the placement section 30. The placement section 30 is configured as a paper box having a substantially cuboidal shape with open upper face and lower face. The four side faces of the placement section 30 configure an external wall section 34 that is vertically upstanding. A placement frame 32 that is a frame in which a smart device 50 (see Fig. 10) described later is to be placed is formed on the upper face of the placement section 30. A light blocking section 33 of a box shape having a closed upper face including an open window 31 and an open lower face (see Fig. 7) is formed on one side (hereafter referred to as the "front side") of the interior of the placement section 30.

Herein, the four faces of the external wall section 34 include a front face 34a that is a face on which the light blocking section 33 is positioned, a back face 34b that is a face on an opposite side to the front face 34a, a left side face 34c that is a face on a left side when viewed from the front face 34a, and a right side face 34d that is a face on an opposite side to the left side face 34c. The interior of the placement section 30 is partitioned by a reinforcement portion 35 that is parallel to the front face 34a and the back face 34b. A rectangular shaped cut-out portion 36 is also formed at a lower edge of the front side of the left side face 34c.

### (4) Housing

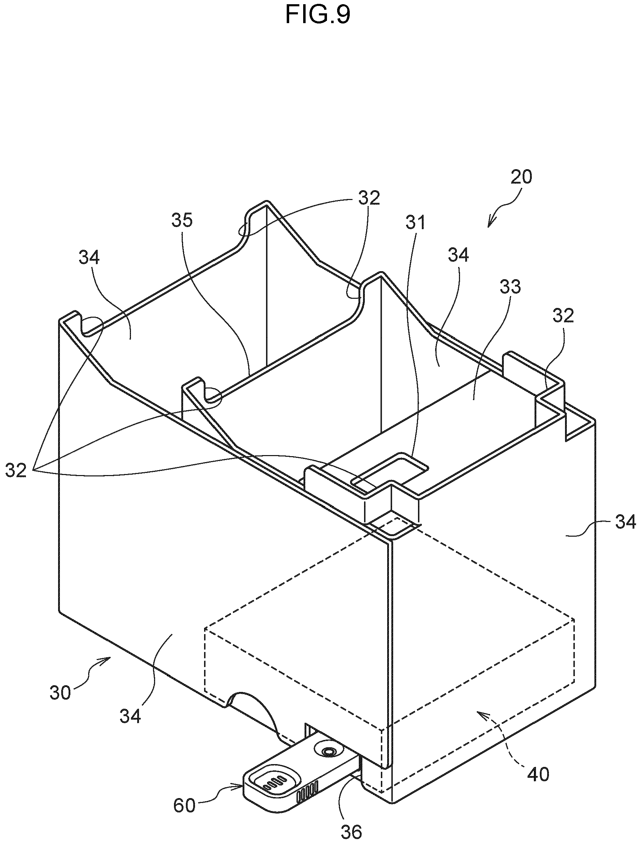

As illustrated in Fig. 7, there is a gap between the lower edge of the light blocking section 33 and the lower edge of the external wall section 34, and a space surrounded on four sides by the front face 34a, the reinforcement portion 35, the left side face 34c, and the right side face 34d, and having a height of this gap is called a housing area 37. Assembly of the housing 20 illustrated in Fig. 8 is completed by the holder 40 being assembled to the housing area 37. In such a state, the cut-out portion 36 of the placement section 30 and the insertion port 41 of the holder 40 are aligned with each other. A state in which the test strip 60 is mounted to the insertion port 41 as illustrated in Fig. 4 and Fig. 5 in this state is as illustrated by the upper perspective view of Fig. 9.

### (5) Smart Device

Fig. 10 is a bottom view of the smart device 50 when employed in the measurement system 10 of the present exemplary embodiment (see Fig. 11) in a state placed in the housing 20. Although, in the present exemplary embodiment, a smartphone is employed as the smart device 50, a tablet terminal with a camera function may be employed as the smart device 50. An imaging section 51 configured by (comprising) a camera, and an illuminator 52 configured by (comprising) a flash that radiates visible light in cooperation therewith, are provided at a bottom face side (so-called back face) of the smart device 50. The imaging section 51 acquires optical information of the test strip 60 irradiated by the light source 42 as an image. Note that the top face side (so-called front face) of the smart device 50 is configured by a display 53.

### (6) Measurement System

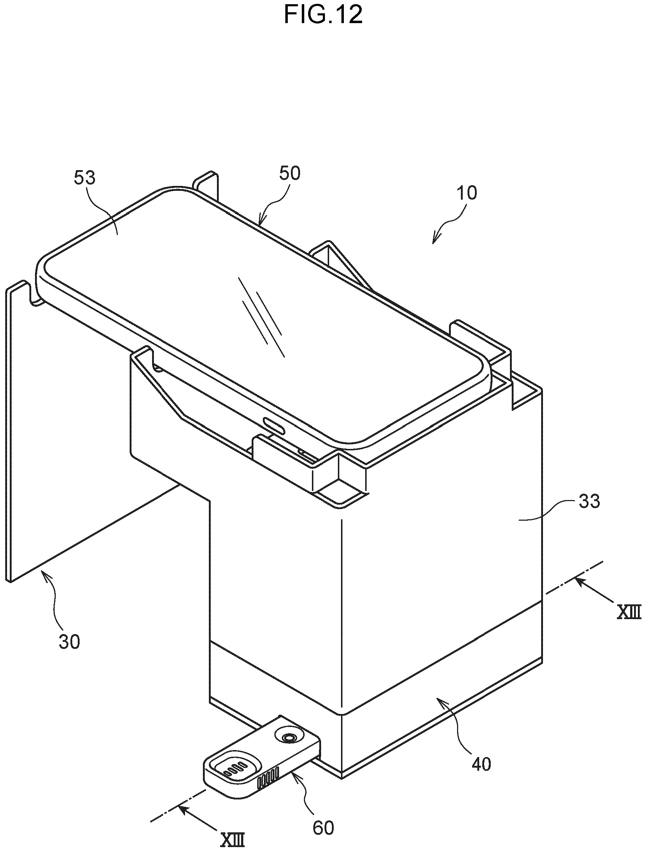

At the inside of the placement frame 32 of the housing 20 illustrated in Fig. 9, the smart device 50 illustrated in Fig. 10 is placed with the display 53 facing upward while the imaging section 51 and the illuminator 52 are aligned with the window 31, with the measurement system 10 of the present exemplary embodiment configured as illustrated in the upper perspective view of Fig. 11. Entry of external light is impeded when, from this state, the measurement opening section 43 and the identification opening section 44 of the holder 40 are covered by the light blocking section 33, as illustrated in the upper perspective view of Fig. 12, which illustrates a state in which the front face 34a, the left side face 34c, and the right side face 34d of the external wall section 34 is supposed to be removed.

Moreover, as illustrated in Fig. 13, which illustrates a cross-section taken along line XIII-XIII of Fig. 12, the measurement opening section 43 and the optical filter 45 are positioned above the measurement region 61 of the test strip 60, and the identification opening section 44 is positioned above the identification region 62. Furthermore, the light source 42 is installed below and slightly at the back face side of the identification opening section 44 and radiates from diagonally above the measurement region 61. The window 31 of the placement section 30 is positioned directly above the measurement opening section 43, and, as well as the measurement region 61, the identification region 62 is also contained in the visual field of the imaging section 51 of the smart device 50.

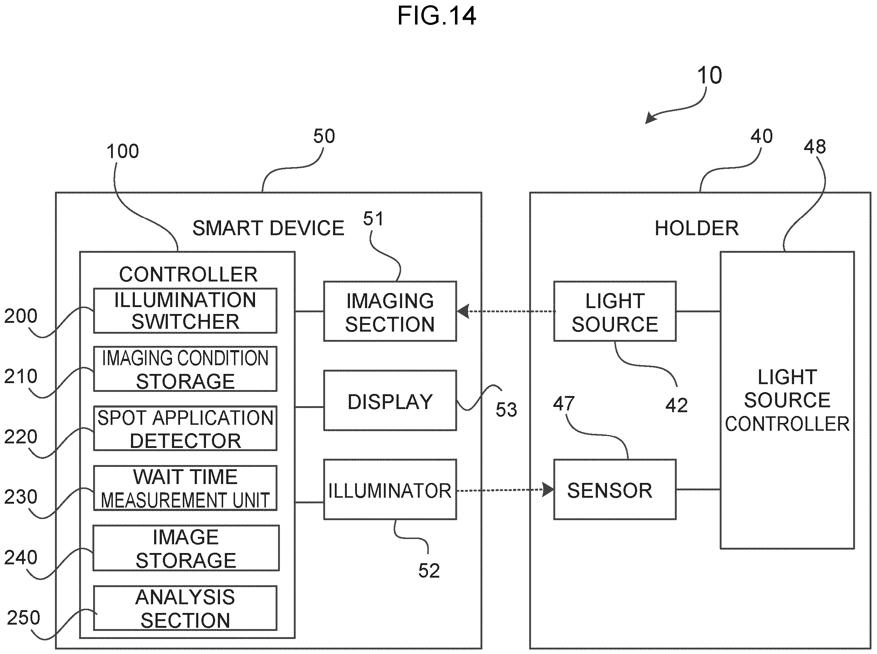

Fig. 14 illustrates the measurement system 10 of the present exemplary embodiment as a functional block diagram. The smart device 50 is provided with the imaging section 51 and the illuminator 52 illustrated in Fig. 10, and the display 53 illustrated in Fig. 11, and also with a controller 100 that controls these. The controller 100 utilizes a CPU 110, a ROM 120, a RAM 130, and a storage device 150, described later, as hardware resources of a computer to function as each of the following means.

Namely, the controller 100 functions as an illumination switcher 200 that switches illumination by the illuminator 52 ON/OFF (turn-on/turn-off). The illumination switcher 200 may, specifically, be implemented as an application installed on the smart device 50, or alternatively may be implemented as a means that utilizes electrical or optical sensing with the holder 40, or as a wireless communication means (e.g., Bluetooth (registered trademark) or the like) with the holder 40. Moreover, the controller 100 functions as an imaging condition storage 210 stored with conditions for capturing with the imaging section 51. Conditions defined as the imaging conditions include, for example, a wait time needed for reaction between the measurement target and the reagent. Moreover, through the imaging section 51, the controller 100 functions as a spot application detector 220 to detect spot application of the sample to the test strip 60. Moreover, the controller 100 also functions as a wait time measurement unit 230 to measure the wait time. Then, the controller 100 functions as an image storage 240 to store an image of a measurement region 61 captured by the imaging section 51. Furthermore, the controller 100 functions as an analysis section 250 that analyzes an image captured by the imaging section 51 as optical information.

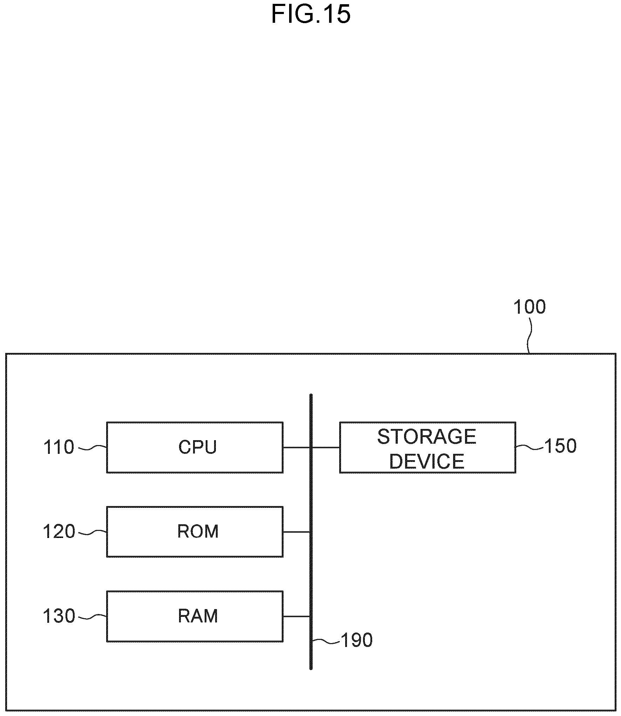

As illustrated by the hardware configuration of Fig. 15, the controller 100 includes the central processing unit (CPU) 110, the read only memory (ROM) 120, the random access memory (RAM) 130, and the storage device 150. These configurations are each connected together through a bus 190 so as to be capable of communicating with each other.

The CPU 110 is a central processing unit that executes various programs and controls each section. Namely, the CPU 110 reads a program from the ROM 120 or the storage device 150, and executes the program using the RAM 130 as a work area. The CPU 110 controls the measurement system 10 according to the program recorded on the ROM 120 or the storage device 150.

The ROM 120 is stored with various programs and various data. The RAM 130 serves as a work area for temporarily storing programs and/or data. The storage device 150 is configured as storage by a hard disk drive (HDD), solid state drive (SSD), flash memory, or the like, and stores various programs including an operating system and various data.

On the other hand, the holder 40 includes the light source 42 that radiates the measurement region 61, the sensor 47 that detects ON/OFF (turn-on/turn-off) of the illuminator 52, and a light source controller 48 that turns the light source 42 on when input with a signal from the sensor 47. The light source controller 48 is configured by hardware resources of a computer similarly to the controller 100 of the smart device 50. Note that, as long as the light source controller 48 is able to control to turn the light source 42 on when capturing the measurement region 61 described later, the light source controller 48 may be implemented to turn the light source 42 on irrespective of input mode (e.g., wired or wireless) of the signal from the sensor 47. Moreover, the light source controller 48 can control to turn the light source 42 off. Herein, the light source 42 is a light source that radiates light of a predetermined wavelength appropriate for detecting the measurement target onto the measurement region 61. For example, appropriate capturing can be performed by making the light source 42 ultraviolet light in cases in which a substance that reacts with the measurement target in the target reaction band 70 of the measurement region 61 absorbs ultraviolet light. Note that, thereby, the light source 42 with a wavelength corresponding to the measurement target is accordingly employed in capturing of the measurement region 61, enabling the illuminator 52 of the smart device 50 be used as a light source in capturing the identification region 62.

As described above, the measurement system 10 of the present exemplary embodiment includes the test strip 60 that includes the introduction portion 63 at the upstream side into which the sample is introduced, the measurement portion 72 at the downstream side on which the reaction reagent that reacts with the measurement target contained in the sample is immobilized, and the developing portion 73 for spreading the sample from the introduction portion 63 to the measurement portion 72, the light source 42 that radiates light of a predetermined wavelength onto the test strip 60 into which the sample is introduced, the imaging section 51 acquiring the optical information of the test strip 60 irradiated by the light source 42, and the analysis section 250 that determines a type of the sample from the optical information of the upstream region 74 at the upstream side of the developing portion 73 in the optical information acquired by the imaging section 51.

In the measurement system 10 of the present exemplary embodiment, the analysis section 250 preferably determines the type of the sample based on the optical information of the upstream region 74 with respect to a standard value based on optical information of the standard region 76 (see Fig. 3B) that is a region resulting from removing the target reaction band 70 and the control reaction band 71 from the comparison region 75, which is a region in the developing portion 73 other than the upstream region 74. Herein, the optical information is preferably obtained at each predetermined position along a length direction of the developing portion 73, as an average value of optical intensities at each of predetermined positions along a direction (specifically a width direction of the test strip 60 illustrated in Fig. 3B) orthogonal to this length direction. Moreover, the standard value is preferably an average value of optical information in the standard region 76. Note that the "predetermined positions" referred to herein are preferably defined based on pixels of a photograph captured by the imaging section 51. Furthermore, the predetermined wavelength for the light source 42 to irradiate the test strip 60 is preferably a wavelength absorbed by blood cell components in the sample.

The measurement system 10 of the present exemplary embodiment functions in the following manner. First, light of a predetermined wavelength (preferably ultraviolet light having a wavelength absorbed by blood cell components in a sample) is radiated from the light source onto the test strip 60 into which the sample is introduced. The optical information of the irradiated test strip 60 is acquired by the imaging section 51. The optical information acquired by the imaging section 51 (preferably pixel information of a photograph captured by the imaging section 51) also contains optical information of the upstream region 74. The analysis section 250 determines a type of the sample (e.g., whole blood sample or non-blood cell sample) from the acquired optical information of the upstream region 74. This determination is, preferably, performed based on optical information of the comparison region 75, for example determined based on the optical information of the upstream region 74 with respect to a standard value determined as an average value of the optical information in the comparison region 75 (more preferably an average value of the optical information of the standard region 76).

More specifically, optical information (i.e., pixel information) for each of the predetermined positions in the upstream region 74 is compared against the standard value, and the type of the sample is determined by how much divergence there is from the standard value. For example, in cases in which the sample contains blood cells, a fluorescence intensity generated by the test strip 60 absorbing the irradiated ultraviolet light is lower in a region in which blood cells are suspected to be present (i.e., the upstream region 74) than when the sample does not contain blood cells (i.e., in cases in which the sample is a non-blood cell sample). On the other hand, the fluorescence intensity in the standard region 76 is not affected by whether or not the sample contains blood cells. Therefore, whether the sample is a whole blood sample or a non-blood cell sample can be determined by the degree by which the optical information (i.e., the fluorescence intensity) in each of the predetermined positions in the upstream region 74 is lower than the standard value based on the optical information (i.e., the fluorescence intensity) in the standard region 76.

For example, the sample can be determined as being a whole blood sample in cases in which there is a drop of a predetermined proportion (e.g. 10%) or greater with respect to the standard value in the optical information of each of the predetermined positions in the upstream region 74, and can be determined as being a non-blood cell sample in other cases. Alternatively, the sample can be determined as being a whole blood sample in cases in which there is a predetermined number of positions (e.g. 10 positions) or greater at which the optical information of each of the predetermined positions in the upstream region 74 is lower than the standard value, and can be determined as being a non-blood cell sample in other cases. As another determination method, the sample can be determined as being a whole blood sample in cases in which there is optical information present lower than the standard value in the optical information of each of the predetermined positions in the upstream region 74, and the optical information lower than the standard value is continuous from this point to the upstream side end thereof, and can be determined as being a non-blood cell sample in other cases. As a further alternative method, the sample can be determined as being a whole blood sample in cases in which there is a change ratio of the optical information in the upstream region 74 (i.e., a proportion of the difference between the standard value and the optical information with respect to the standard value) present below the standard value, and there are a continuous predetermined number of positions (e.g., 10 positions) or greater below the standard value from this point in the upstream direction, and can be determined as being a non-blood cell sample in other cases.

Note that, by appropriately adjusting a structure of the test strip 60 (e.g., the characteristics of the development membrane 64A, the sample pad 64B, and the conjugate pad 64C) and the degree of dilution of the sample, a configuration can be achieved in which, in cases in which the sample contains blood cell, the blood cells are stopped in the upstream region 74 of the developing portion 73 in a state in which the sample is spread in the test strip 60.

Moreover, the smart device 50 includes the imaging section 51 and the analysis section 250, as illustrated in Fig. 14. Namely, the smart device 50 of the present exemplary embodiment includes the imaging section 51 that acquires the optical information of the upstream region 74 that is at the upstream side of the developing portion 73 in a state in which light of a predetermined wavelength is irradiated onto the test strip 60 to which the sample is introduced, with the test strip 60 including the introduction portion 63 at the upstream side into which the sample is introduced, including the measurement portion 72 at the downstream side on which the reaction reagent that reacts with a measurement target contained in the sample is placed, and including the developing portion 73 for spreading the sample from the introduction portion to downstream of the measurement portion 72, and the smart device 50 includes the analysis section 250 that analyzes the optical information of the upstream region 74 and determines a type of the sample. Determination of the type of the sample by the analysis section 250 is described above.

The measurement system 10 of the present exemplary embodiment described above is able to execute a measurement method, which includes: a stage that radiating light of a predetermined wavelength onto a test strip 60 to which a sample is introduced, the test strip 60 including the introduction portion 63 at an upstream side into which a sample is introduced, a measurement portion 72 at a downstream side on which a reaction reagent that reacts with a measurement target contained in the sample is placed, and a developing portion 73 for spreading the sample from the introduction portion 63 to downstream of the measurement portion 72; acquiring optical information of the upstream region 74 at the upstream side of the developing portion in the state in which light of the predetermined wavelength is irradiated; and analyzing the acquired optical information of the upstream region 74 and determining a type of the sample.

The measurement method described above can be executed by a measurement program that causes a computer to function as: a unit to radiate light of a predetermined wavelength onto a test strip to which a sample is introduced, the test strip including an introduction portion at an upstream side into which a sample is introduced, a measurement portion at a downstream side on which a reaction reagent that reacts with a measurement target contained in the sample is placed, and a developing portion for spreading the sample from the introduction portion to downstream of the measurement portion; a unit to acquire optical information of the upstream region at the upstream side of the developing portion in the state in which light of the predetermined wavelength is irradiated; and a unit to analyze the acquired optical information and to determine a type of the sample.

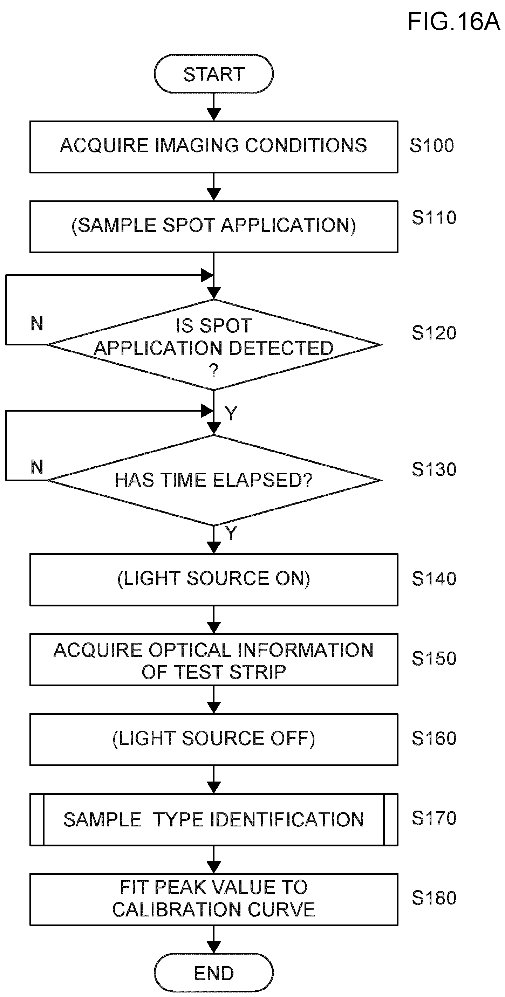

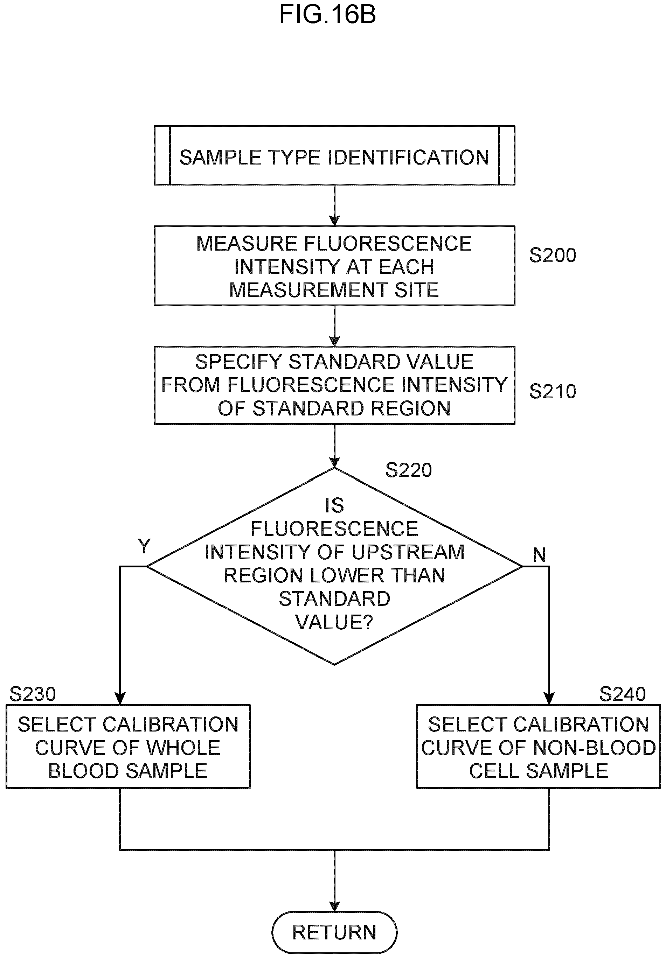

An example of the measurement method of the exemplary embodiment described above will now be described with reference to the flowchart illustrated in Fig. 16A and Fig. 16B. Note that, in this flowchart, stages indicting operations other than actions directly performed by the smart device 50 are indicated in parentheses, with only an outline thereof being shown.

First, as illustrated in Fig. 11, the measurement system 10 is prepared with the smart device 50 placed in the housing 20 with the holder 40 mounted to the placement section 30, and the test strip 60 is inserted through the insertion port 41. At this stage, when a screen of the display 53 (see Fig. 11 and Fig. 14) is operated, a measurement application installed on the smart device 50 is started up, and measurement is started.

First, at the stage indicated by S100, the imaging section 51 captures an image of the identification region 62 (see Fig. 3A) in a state in which the illuminator 52 is turned on, and based on this, the controller 100 references the imaging condition storage 210 (see Fig. 14) and acquires imaging conditions for measuring with the test strip 60. Details regarding this stage are omitted.

Next, at the stage illustrated in S110, an appropriate amount of a test sample is spotted onto the introduction portion 63 (see Fig. 3B) of the test strip 60 by a measurer. The spotted test sample is spread toward the downstream side by the test paper 64 (see Fig. 3B) inside the test strip 60.

Meanwhile, at the stage indicated by S120, the spot application detector 220 (see Fig. 14) of the controller 100, through the image from the imaging section 51, continues to monitor whether or not an image is detected to indicate that spot application is completed (e.g., that the control reaction band 71 is generated due to a reaction with the measurement target in the measurement region 61 (see Fig. 3B)). Processing proceeds to the stage indicated by S130 when such an image is detected.

At the stage indicated by S130, after detection of completion of spot application at the stage indicated by S120, the wait time measurement unit 230 (see Fig. 14) of the controller 100 continues to monitor whether or not a wait time needed for reaction between the measurement target and the reagent in the test paper 64 is elapsed, from among the imaging conditions acquired at the stage indicated by S100.

When determination by the wait time measurement unit 230 that the wait time is elapsed at the stage indicated by S130, the illumination switcher 200 turns the illuminator 52 off and turns the light source 42 on at the stage indicated by S140.

When the light source 42 is turned on, at the stage indicated by S150, the imaging section 51 captures the developing portion 73 (see Fig. 3B) containing the target reaction band 70 visualized by the wavelength of the measurement light from the light source 42 in the measurement region 61, and acquires this as optical information of the test strip 60. When the acquisition of the optical information is completed, at the stage indicated by S160, the light source controller 48 of the holder 40 turns the light source 42 off. Then at the stage indicated by S170, identification of the sample type is executed.

Fig. 16B is a flowchart illustrating details of sample type identification of the stage indicated by S170. First, at the stage indicated by S200, the analysis section 250 measures the fluorescence intensity at each measurement site from the optical information of the test strip 60 acquired by the imaging section 51 at the stage indicated by S150. Herein, the optical information of the test strip 60 is actually the image information of the developing portion 73 illustrated in Fig. 3B. This image information is a collection of pixels with a length direction of the test strip 60 as an X axis and a width direction thereof as a Y axis, with information of light generation intensity, color, and the like associated with each pixel.

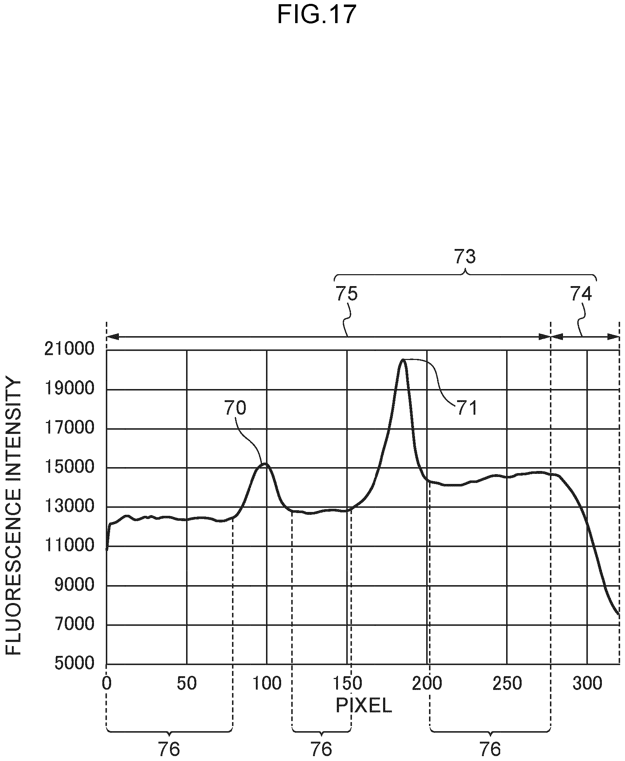

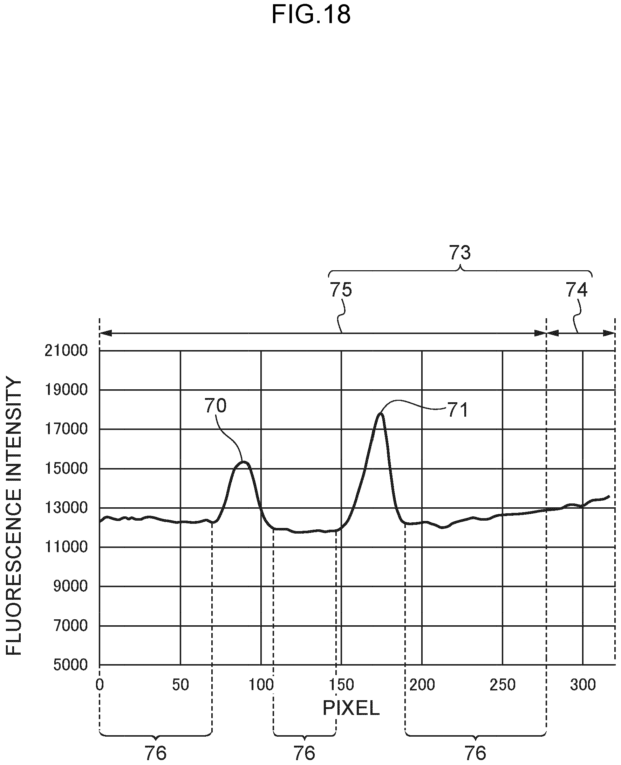

Fig. 17 and Fig. 18 are graphs of the fluorescence intensity for each of the positions in the length direction in the image information of the developing portion 73 for a case in which whole blood and blood plasma are respectively spotted as the sample on the test strip 60. The horizontal axis in both diagrams represents the X axis, and specifically represents a pixel number of positions on the image information in the length direction of the developing portion. Note that, in both diagrams smaller pixel numbers indicate the downstream side, and larger pixel numbers indicate the upstream side. Moreover, the vertical axis in each of the diagrams expresses an average value of the fluorescence intensity along the Y axis. In both diagrams a peak from the target reaction band 70 caused by the measurement target appears at the downstream side (left side in the diagram), and a peak from the control reaction band 71 appears at the upstream side. Furthermore, whereas there is a drop in the fluorescence intensity seen at positions corresponding to the upstream region 74 in Fig. 17, there is no drop in the fluorescence intensity seen at positions corresponding to the upstream region 74 in Fig. 18.

Then, after the fluorescence intensity is measured for each of the measurement sites at the stage indicated by S200, at the stage indicated by S210 the analysis section 250 specifies the standard value from the fluorescence intensity of the standard region 76 that is a region in the comparison region 75 other than the target reaction band 70 and the control reaction band 71. Namely, the standard value is specified based on the fluorescence intensity corresponding to the standard region 76 as illustrated in Fig. 17 and Fig. 18. The standard value may, for example, be an average value of the fluorescence intensity of the standard region 76. Alternatively, a minimum fluorescence intensity in the standard region 76 may be employed as the standard value.

After the standard value is specified at the stage indicated by S210, at the stage indicated by S220, the analysis section 250 determines whether or not the fluorescence intensity of the upstream region 74 is lower than the standard value. For example, the fluorescence intensity of the upstream region 74 can be determined to be lower than the standard value when the fluorescence intensities at the predetermined positions in the upstream region 74 which are lower than the standard value occupy a predetermined proportion (e.g., 10%) or greater. Alternatively, the fluorescence intensity of the upstream region 74 can be determined to be lower than the standard value when the fluorescence intensities at the predetermined positions in the upstream region 74 which are lower than the standard value occupy a predetermined number (e.g., ten) or greater. Alternatively, the sample may be determined to be a whole blood sample in cases in which, from among the fluorescence intensities of the predetermined positions in the upstream region 74, a fluorescence intensity lower than the standard value is present at one position, and fluorescence intensities lower than the standard value continue from this position to the upstream side end position, and otherwise the sample can be determined to be a non-blood cell sample. Alternatively, the sample can be determined to be a whole blood sample in cases in which a change ratio of the fluorescence intensity in the upstream region 74 (i.e., a proportion of the difference between the standard value and the fluorescence intensity with respect to the standard value) lower than the standard value is present at one position, and the change ratios at predetermined number (e.g., ten) or more of consecutive upstream positions from the position are lower than the standard value, and otherwise the sample can be determined to be a non-blood cell sample. For example, as illustrated in Fig. 17, the analysis section 250 determines that the sample is a whole blood sample in cases in which a fluorescence intensity lower than the standard value is recognized in the upstream region 74. On the other hand, as illustrated in Fig. 18, the analysis section 250 determines that the sample is a non-blood cell sample in cases in which fluorescence intensity lower than the standard value is not recognized in the upstream region 74.

Herein, the storage device 150 of the controller 100 is provided in advance with information about a calibration curve obtained by measuring a whole blood sample containing a measurement target at a known concentration, and information about a calibration curve obtained by measuring a non-blood cell sample (e.g., a blood plasma sample) containing a measurement target at a known concentration. Then, in cases in which the fluorescence intensity of the upstream region 74 is determined to be lower than the standard value at the stage indicated by S220, the analysis section 250 selects the calibration curve of the whole blood sample stored in the storage device 150 at the stage indicated by S230. On the other hand, in cases in which the fluorescence intensity of the upstream region 74 is not determined to be lower than the standard value at the stage indicated by S220, the analysis section 250 selects the calibration curve of the non-blood cell sample stored in the storage device 150 at the stage indicated by S240.

Then, In cases in which either calibration curve is selected, at the stage indicated by S180 of Fig. 16A, the analysis section 250 measures a concentration of the measurement target in the sample by fitting a peak value (i.e., fluorescence intensity) of the target reaction band 70 to the selected calibration curve.

Thereby, according to the measurement system 10 and the measurement method of the present exemplary embodiment, it is possible to measure a measurement target in a sample using an appropriate calibration curve as long as the sample is similarly spotted onto the test strip 60 even in cases in which it is unclear in advance as to whether or not the sample is a whole blood sample or a non-blood cell sample.

Note that samples that may be measured by the measurement system 10 and the measurement method of the present exemplary embodiment are not limited to a whole blood sample or a non-blood cell sample as described above. For example, even in cases in which it is unclear whether or not a particular particle shaped substance is contained in a sample suspected of containing a measurement target, the measurement target in the sample can be measured similarly to the aforementioned by storing information of calibration curves related to concentrations of the measurement target for a sample containing such a particle shaped substance, and for a sample not containing such a particle shaped substance, in advance in the storage device 150.

### Examples

Hereinafter, examples of test strips configured so as to confine blood cell components to the upstream region 74 in a state in which a whole blood sample that is spotted onto the test strip 60 spreads through the test paper 64 under the following specific conditions were examined. In the present examples, test strips 60 configured as illustrated in Fig. 3B and Fig. 3C were employed, and the sizes of portions indicated in Fig. 3B and Fig. 3C were as listed in the Table 1 shown below.

**Table 1**

| Site | Size (mm) |
|---|---|
| α | 15 |
| β | 17.5 |
| γ | 25 |
| δ | 10 |
| ε | 17 |
| ζ | 22 |
| η | 5 |
| θ | 64.7 |
| 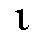 | 2 |
| κ | 2.3 |
| λ | 20.7 |

Namely, in the test strips 60 of the present examples, a length of the developing portion 73 was λ (20.7 mm) shown above.

Moreover, four types of membranes for immunochromatography, IAB090, IAB120, IAB135, and IAB180 (all from ADVANTEC), were employed as the development membrane 64A of the test strip 60. The liquid permeation speeds for 4 cm lengths thereof were 90 seconds, 120 seconds, 135 seconds, and 180 seconds, respectively.

As the sample to be spotted onto the test strip 60, whole blood of the respective amounts of the Table 2 shown below was adjusted by being suspended in 130 µL of a diluent (specifically, a buffer solution containing a surfactant, a sugar, and a blocking agent such as bovine serum albumin). A 90 µL sample thereof was then spotted onto the introduction portion 63 of the test strip 60 mounted with the four types of development membrane 64A shown above, and the results of developing are illustrated in the following Table 2. The evaluation results illustrated in the following Table 2 are as listed below.
A: no effect on the measurement, and possible to identify the sample type.
B: Possible to identify the sample type by changing the shape of the measurement strip.
C1: Likely to affect on the measurement.
C2: Impossible to identify the sample type.

**Table 2**

| Whole Blood Amount (µL/ 130 µL diluent) | Permeation Speed of Development Membrane (s/4 cm) | | | |
|---|---|---|---|---|
| | 90 | 120 | 135 | 180 |
| 15 | C1 | A | A | B |
| 12 | C1 | A | A | B |
| 10 | C1 | A | A | B |
| 7 | A | A | B | B |
| 5 | A | B | B | C2 |
| 3 | B | B | C2 | C2 |
| 1 | C2 | C2 | C2 | C2 |

First, it is apparent, from combinations evaluated as "A" in Table 2 shown above, that, in the test strips 60 of the present examples having the sizes illustrated in Table 1, the present examples were appropriate because the blood cell component could be confined to the upstream region 74. Moreover, combinations evaluated as "B" in Table 2 shown above, although not being applicable as the test strips 60 of the present example, may conceivably confine blood cell components to the upstream region 74 by changing the sizes of each portion of the test strip 60.

On the other hand, combinations evaluated as "C1" were determined to impede detection of the target reaction band 70 and were unsuitable due to blood cell components exceeding the upstream region 74 to reach the measurement portion 72. Moreover, combinations evaluated as "C2" were determined to not enable identification of sample type due to the blood cell components not reaching the developing portion 73.

Thereby, blood cells contained in the samples are able to be confined to the upstream region of the developing portion by employing a membrane having a desired permeation speed as the nitrocellulose membrane as the development membrane, and by appropriately adjusting dilution of the whole blood sample, and furthermore the shape of the test strip.

### Preferable Embodiments

The following clauses set out features of the invention which may not presently be claimed in this application, but which may form the basis for future amendment or a divisional application.
<1> A measurement system including:
   a test strip including an introduction portion at an upstream side into which a sample is introduced, a measurement portion at a downstream side on which a reaction reagent that reacts with a measurement target contained in the sample is immobilized, and a developing portion for spreading the sample from the introduction portion to downstream of the measurement portion;
   a light source radiating light of a predetermined wavelength onto the test strip into which the sample is introduced;
   an imaging section acquiring optical information of the test strip irradiated by the light source; and
   an analysis section determining a type of the sample from the optical information of an upstream region located at an upstream side in the developing portion from among the optical information acquired by the imaging section.
<2> The measurement system of <1>, wherein the optical information is obtained at each predetermined position along a length direction of the developing portion as an average value of optical information for each of predetermined positions along a direction orthogonal to the length direction.
<3> The measurement system of <2>, wherein the predetermined positions are determined based on pixels of a photograph captured by the imaging section.
<4> The measurement system of any one of <1> to <3>, wherein the analysis section determines the type of the sample based on optical information of the upstream region with respect to a standard value determined based on optical information in a comparison region that is a region in the developing portion other than the upstream region.
<5> The measurement system of <4>, wherein:
   the comparison region includes a region at which a target reaction band appears, generated by a specific reaction with the measurement target contained in the sample, and a region at which a control reaction band appears, generated by a non-specific reaction with the sample; and
   the standard value is determined based on optical information in a standard region that is a region in the comparison region other than the region at which the target reaction band appears and the region at which the control reaction band appears.
<6> The measurement system of <5>, wherein the standard value is an average value of the optical information in the standard region.
<7> The measurement system of <6>, wherein optical information at each predetermined position along a length direction of the developing portion in the upstream region is compared against the standard value.
<8> The measurement system of <7>, wherein the type of the sample is determined by whether or not the optical information at each of the predetermined positions in the upstream region contains more than a predetermined proportion of values lower than the standard value.
<9> The measurement system of <7>, wherein the type of the sample is determined by whether or not in the optical information at each of the predetermined positions in the upstream region contains more than a predetermined number of values lower than the standard value.
<10> The measurement system of any one of <1> to <9>, wherein the predetermined wavelength is a wavelength absorbed by blood cell components in the sample.
<11> A measurement method including:
   radiating light of a predetermined wavelength onto a test strip including an introduction portion at an upstream side into which a sample is introduced, a measurement portion at a downstream side on which a reaction reagent that reacts with a measurement target contained in the sample is immobilized, and a developing portion for spreading the sample from the introduction portion to downstream of the measurement portion;
   acquiring optical information of the irradiated test strip; and
   determining type of the sample from the optical information of an upstream region located at an upstream side in the developing portion from among the acquired optical information.
<12> The measurement method of <11>, wherein the optical information is obtained at each predetermined position along a length direction of the developing portion as an average value of optical information for each of predetermined positions along a direction orthogonal to the length direction.
<13> The measurement method of <12>, wherein the predetermined positions are determined based on pixels of a photograph captured by the imaging section.
<14> The measurement method of any one of <11> to <13>, wherein, in determining the type of the sample, the type of the sample is determined based on optical information of the upstream region with respect to a standard value determined based on optical information in a comparison region that is a region in the developing portion other than the upstream region.
<15> The measurement method of <14>, wherein:
   the comparison region includes a region at which a target reaction band appears, generated by a specific reaction with the measurement target contained in the sample, and a region at which a control reaction band appears, generated by a non-specific reaction with the sample; and
   the standard value is determined based on optical information in a standard region that is a region in the comparison region other than the region at which the target reaction band appears and the region at which the control reaction band appears.
<16> The measurement method of <15>, wherein the standard value is an average value of the optical information in the standard region.
<17> The measurement method of <16>, wherein the optical information at each predetermined position along a length direction of the developing portion in the upstream region is compared against the standard value.
<18> The measurement method of <17>, wherein the type of the sample is determined by whether or not the optical information at each of the predetermined positions in the upstream region contains more than a predetermined proportion of values lower than the standard value.
<19> The measurement method of <17>, wherein the type of the sample is determined by whether or not the optical information at each of the predetermined positions in the upstream region contains more than a predetermined number of values lower than the standard value.
<20> The measurement method of any one of <11> to <19>, wherein the predetermined wavelength is a wavelength absorbed by blood cell components in the sample.
<21> A computer-readable medium storing a measurement program that causes a computer to function as:
   a unit to radiate light of a predetermined wavelength onto a test strip including an introduction portion at an upstream side into which a sample is introduced, a measurement portion at a downstream side on which a reaction reagent that reacts with a measurement target contained in the sample is immobilized, and a developing portion for spreading the sample from the introduction portion to downstream of the measurement portion;
   a unit to acquire optical information of the irradiated test strip; and
   a unit to determine type of the sample from the optical information of an upstream region located at an upstream side in the developing portion from among the acquired optical information.
<22> The computer-readable medium of <21>, wherein the optical information is obtained at each predetermined position along a length direction of the developing portion as an average value of optical information for each of predetermined positions along a direction orthogonal to the length direction.
<23> The computer-readable medium of <22>, wherein the predetermined positions are determined based on pixels of a photograph captured by the imaging section.
<24> The computer-readable medium of any one of <21> to <23>, wherein the unit to determine the type of the sample determines the type of the sample based on optical information of the upstream region with respect to a standard value determined based on optical information in a comparison region that is a region in the developing portion other than the upstream region.
<25> The computer-readable medium of <24>, wherein:
   the comparison region includes a region at which a target reaction band appears, generated by a specific reaction with the measurement target contained in the sample, and a region at which a control reaction band appears, generated by a non-specific reaction with the sample; and
   the standard value is determined based on optical information in a standard region that is a region in the comparison region other than the region at which the target reaction band appears and a region at which the control reaction band appears.
<26> The computer-readable medium of <25>, wherein the standard value is an average value of the optical information in the standard region.
<27> The computer-readable medium of <26>, wherein optical information at each predetermined position along a length direction of the developing portion in the upstream region is compared against the standard value.
<28> The computer-readable medium of <27>, wherein the type of the sample is determined by whether or not the optical information at each of the predetermined positions in the upstream region contains more than a predetermined proportion of values lower than the standard value.
<29> The computer-readable medium of <27>, wherein the type of the sample is determined by whether or not in the optical information at each of the predetermined positions in the upstream region contains more than a predetermined number of values lower than the standard value.
<30> The measurement program of any one of <21> to <29>, wherein the predetermined wavelength is a wavelength absorbed by blood cell components in the sample.
<31> A smart device including:
   an imaging section acquiring optical information of a test strip that includes an introduction portion at an upstream side into which a sample is introduced, a measurement portion at a downstream side on which a reaction reagent that reacts with a measurement target contained in the sample is immobilized, and a developing portion for spreading the sample from the introduction portion to downstream of the measurement portion, by acquisition in a state in which light of a predetermined wavelength is being radiated onto the test strip; and
   an analysis section determining a type of the sample from the optical information of an upstream region located at an upstream side in the developing portion from among the optical information acquired by the imaging section.
<32> The smart device of <31>, wherein the optical information is obtained at each predetermined position along a length direction of the developing portion as an average value of optical information for each of predetermined positions along a direction orthogonal to the length direction.
<33> The smart device of <32>, wherein the predetermined positions are determined based on pixels of a photograph captured by the imaging section.
<34> The smart device of any one of <31> to <33>, wherein the analysis section determines the type of the sample based on optical information of the upstream region with respect to a standard value determined based on optical information in a comparison region that is a region in the developing portion other than the upstream region.
<35> The smart device of <34>, wherein:
   the comparison region includes a region at which a target reaction band appears, generated by a specific reaction with the measurement target contained in the sample, and a region at which a control reaction band appears, generated by a non-specific reaction with the sample; and
   the standard value is determined based on optical information in a standard region that is a region in the comparison region other than the region at which the target reaction band appears and a region at which the control reaction band appears.
<36> The smart device of <35>, wherein the standard value is an average value of the optical information in the standard region.
<37> The smart device of <36>, wherein optical information at each predetermined position along a length direction of the developing portion in the upstream region is compared against the standard value.
<38> The smart device of <37>, wherein the type of the sample is determined by whether or not the optical information at each of the predetermined positions in the upstream region contains more than a predetermined proportion of values lower than the standard value.
<39> The smart device of <37>, wherein the type of the sample is determined by whether or not the optical information at each of the predetermined positions in the upstream region contains more than a predetermined number of values lower than the standard value.
<40> The smart device of any one of <31> to <39>, wherein the predetermined wavelength is a wavelength absorbed by blood cell components in the sample.

### Industrial Applicability

The present invention is applicable to a system and measurement instrument for measuring a measurement target in a sample using a test strip.

## Claims

1. A measurement system (10) comprising:
a test strip (60) including an introduction portion (63) at an upstream side into which a sample is introduced, a measurement portion (72) at a downstream side on which a reaction reagent that reacts with a measurement target contained in the sample is immobilized, and a developing portion (73) for spreading the sample from the introduction portion (63) to downstream of the measurement portion (72);
a light source (42) configured to radiate light of a predetermined wavelength onto the test strip (60) into which the sample is introduced;
an imaging section (51) configured to acquire optical information of the test strip (60) irradiated by the light source (42); and
an analysis section (250) configured to determine a type of the sample from optical information of an upstream region (74) located at an upstream side in the developing portion (73) from among the optical information acquired by the imaging section (51).

2. The measurement system (10) of claim 1, wherein the optical information is obtained at a plurality of predetermined positions along a length direction of the developing portion (73) as an average value of optical information for each of the predetermined positions along a direction orthogonal to the length direction.

3. The measurement system (10) of claim 1 or claim 2, wherein the analysis section (250) is configured to determine the type of the sample based on optical information of the upstream region (74) with respect to a standard value determined based on optical information in a comparison region (75) that is a region in the developing portion (73) other than the upstream region (74).

4. The measurement system (10) of claim 3, wherein:
the comparison region (75) includes a region at which a target reaction band (70) appears, generated by a specific reaction with the measurement target contained in the sample, and a region at which a control reaction band (71) appears, generated by a non-specific reaction with the sample; and
the standard value is determined based on optical information in a standard region (76) that is a region in the comparison region (75) other than the region at which the target reaction band (70) appears and the region at which the control reaction band (71) appears.

5. The measurement system (10) of claim 4, wherein the standard value is an average value of the optical information in the standard region (76).

6. The measurement system (10) of claim 4 or 5, wherein optical information at each predetermined position along a length direction of the developing portion (73) in the upstream region (74) is compared against the standard value.

7. The measurement system (10) of claim 6, wherein the type of the sample is determined by whether or not the optical information at each of the predetermined positions in the upstream region (74) contains more than a predetermined proportion of values lower than the standard value.

8. The measurement system (10) of claim 6, wherein the type of the sample is determined by whether or not the optical information at each of the predetermined positions in the upstream region (74) contains more than a predetermined number of values lower than the standard value.

9. The measurement system (10) of claim 2 or any of claims 3 to 8 when dependent from claim 2, wherein the predetermined positions are determined based on pixels of a photograph captured by the imaging section (51).

10. The measurement system (10) of any of claims 1 to 9, wherein the predetermined wavelength is a wavelength absorbed by blood cell components in the sample.

11. A measurement method comprising:
radiating light of a predetermined wavelength onto a test strip (60) including an introduction portion (63) at an upstream side into which a sample is introduced, a measurement portion (72) at a downstream side on which a reaction reagent that reacts with a measurement target contained in the sample is immobilized, and a developing portion (73) for spreading the sample from the introduction portion (63) to downstream of the measurement portion (72);
acquiring optical information of the irradiated test strip (60); and
determining a type of the sample from optical information of an upstream region (74) located at an upstream side in the developing portion (73) from among the acquired optical information.

12. The measurement method of claim 11 comprising the use of the measurement system (10) of any of claims 1 to 10.
